# EUROPEAN PATENT SPECIFICATION

(11) **EP 3 796 879 B1**
(45) Date of publication and mention of the grant of the patent: **12.11.2025**
(21) Application number: 19807954.3
(22) Date of filing: 22.05.2019
(51) Int. Cl.: A61F 5/455, A61F 5/44, A61M 25/00, A61F 5/453

(54) **EXTERNAL-CATHETER KITS WITH STEP-BY-STEP INSTRUCTIONS**
AUSSENKATHETER-KITS MIT SCHRITTWEISER ANLEITUNG
KITS DE CATHÉTERS EXTERNES AVEC INSTRUCTIONS PAS À PAS

(30) Priority: 23.05.2018 US 201862675633 P; 21.06.2018 US 201862688186 P
(43) Date of publication of application: 31.03.2021
(73) Proprietor: C. R. Bard, Inc., Franklin Lakes, NJ 07417 (US)
(72) Inventor: CHALLA, Pranav, Atlanta, GA 30308 (US); SKELTON, Sarah, Atlanta, GA 30312 (US); LAMBERT, Michael, Cincinnati, OH 45245 (US)
(74) Representative: Hoffmann Eitle
(86) International application number: PCT/US2019/033610
(87) International publication number: WO 2019/226826

(56) References cited:
- GB-A- 2 545 967
- US-A- 5 263 947
- US-A1- 2001 041 882
- US-A1- 2010 331 798
- US-A1- 2011 152 802
- US-A1- 2013 245 496
- US-A1- 2014 039 349
- US-A1- 2014 231 288
- US-A1- 2016 228 676
- US-A1- 2016 228 676
- US-A1- 2016 374 848
- US-A1- 2017 202 699
- US-B1- 6 926 708
- US-B1- 7 588 560
- US-B2- 9 078 760
- "Urocare Urinary Drainage Bottle", UROCARE, 2017, XP055655647, Retrieved from the Internet <URL:https://www.urocare.com/EN/Products/4100EN.php>
- "EXPRESS MEDICAL SUPPLY, INC.", STERIGEAR - 2000ML URINARY DRAINAGE BAG WITH FIG LEAF COVER, 2016, XP055762397, Retrieved from the Internet <URL:https://www.exmed.net/p-3770-sterigear-2000ml-urinary-drainage-bag-with-fig-leaf-cover.aspx>

## Description

### PRIORITY

This application claims the benefit of priority to U.S. Provisional Application No. 62/688,186, filed June 21, 2018, and to U.S. Provisional Application No. 62/675,633, filed May 23, 2018.

### BACKGROUND

Hospitals around the country are understandably focusing their efforts on reducing risk of catheter-associated urinary tract infections ("CAUTIs") in response to at least large penalties put in place by the Affordable Care Act ("ACA"). Steps taken to reduce the risk of such indwelling catheter-associated infections include implementation of a variety of prevention methodologies recommended by the Centers for Disease Control and Prevention ("CDC"), as well as decreasing utilization of indwelling catheters through use of non-indwelling catheter alternatives. With the use of non-indwelling catheter alternatives such as external catheters expected to rise, problems associated with application and management of such catheters once applied are also expected to rise.
US 5,263,947 relates to an external incontinence device comprising a housing having an outlet conduit, a plurality of leaves made of a thin, vapor permeable film connected to the housing, a vapor permeable adhesive on the interior surface of the leaves, a microbial-barrier layer provided on an inner surface of the housing and a barrier disc provided on the inner surface of the housing. US 2016/0228676 relates to a multi-compartment catheterization package, in which a swab is held in an inclined position and the catheterization instructions are imprinted directly on the catheterization tray. US 2001/0041882 relates to a female inter-labial catheter for urinary relief. US 9,078,760 relates to a cover for concealing and supporting a urinary incontinence bag. GB 2 545 967 relates to a urine collection sheath.

Disclosed herein are external catheters, external catheter-related components, and external-catheter kits with step-by-step instructions, providing a standardized system for application and management of such catheters.

### SUMMARY

The invention is defined by the claims. Disclosed herein is an external-catheter kit including one or more external catheters, wherein the one or more external catheters are female external catheters ("FECs"), wherein the FEC comprises an elongate body and gauze covering an aperture in said elongate body, wherein said aperture is configured for fluid connection to a urine-drainage tubing, wherein said urine-drainage tubing is configured for connection to a urine-drainage tubing of a FEC urine-drainage system. The external-catheter kit further includes a cleansing kit, and step-by-step instructions for using the external-catheter kit. The cleansing kit includes one or more individually wrapped towelettes for at least anatomical cleansing before application of any external catheter of the one or more external catheters. The step-by-step instructions for using the external-catheter kit include step- by-step instructions for the anatomical cleansing, external-catheter application, and external-catheter management subsequent to the external-catheter application.

In some embodiments, the external-catheter kit further includes a urine-drainage system. The urine-drainage system includes a urine-collection receptacle, urine-drainage tubing, and one or more connectors to fluidly connect an external catheter of the one or more external catheters to the urine-collection device.

In some embodiments, the urine-drainage system is at least partially preassembled in the external-catheter kit to minimize misconnections and misconnection-based contamination. The urine-drainage tubing is fluidly connected to the urine-collection receptacle in the partially preassembled urine-drainage system.

In some embodiments, the one or more FECs are optionally individually wrapped.

In some embodiments, the external-catheter kit further includes an FEC urine-drainage system. The FEC urine-drainage system includes a urine-collection canister, a lid configured to cover the urine-collection canister, urine-drainage tubing, and one or more connectors to fluidly connect an FEC of the one or more FECs to the urine-collection canister.

In some embodiments, the FEC urine-drainage system is at least partially preassembled in the external-catheter kit to minimize misconnections and misconnection-based contamination. The urine-drainage tubing is fluidly connected to the urine-collection canister through the lid covering the urine-collection canister in the partially preassembled FEC urine-drainage system.

In some embodiments, the external-catheter kit further comprises a urine-privacy cover configured to form a sleeve for covering the urine-collection canister. The sleeve has a cut-out window configured to expose graduations on the urine-collection canister through the window of the sleeve while a remainder of the urine-collection canister is covered by the sleeve.

In some embodiments, the external-catheter kit further comprises a visual duration indicator configured to indicate when a predetermined amount of time has elapsed since activation thereof.

In some embodiments, the external-catheter kit further comprises a securement device configured to secure the urine-drainage tubing of the urine-drainage system to a patient's leg or another portion of the patient's body.

The securement device minimizes any longitudinal or transverse movement of the drainage tubing while in use.

In some embodiments, the step-by-step instructions are provided in a booklet having a picture book format, integrated with packaging for the external-catheter kit, optionally, revealed as one or more components of the external-catheter kit are removed from the external-catheter kit, or a combination thereof.

In some embodiments, the packaging includes a wrap of a first sterilizable material around the components of the external-catheter kit or a tray of a second sterilizable material into which the components of the external-catheter kit are placed. The packaging further includes an outer packaging around the wrap or the tray. The outer packaging is configured for sterilizing the components of the external-catheter kit in the wrap or tray within the outer packaging with a sterilizing gas before sealing the external-catheter kit.

These and other features of the concepts provided herein will become more apparent to those of skill in the art in view of the accompanying drawings and following description, which disclose particular embodiments of such concepts in greater detail.

### DRAWINGS

Embodiments falling under the claimed invention are described in figures 2, 4A, 4B, 6A and 6B. The remaining embodiments are considered useful for understanding the invention.
FIG. 1A provides an MEC of a first style.
FIG. IB provides an MEC of a second style.
FIG. IC provides an MEC of a third style.
FIG. ID provides an MEC disposed over a plastic insert.
FIG. 2 provides an FEC in accordance with some embodiments.
FIG. 3A provides a connector assembly for an MEC disposed in a securement device.
FIG. 3B provides a cross-section of a connector assembly for an MEC disposed in a securement device.
FIG. 3C provides a cross-section of a connector assembly for an MEC disposed in a securement device.
FIG. 3D provides a close-up of a portion of the connector assembly of FIG.3B.
FIG. 3E provides a cross-section of a connector assembly for an MEC disposed in a securement device.
FIG. 4A provides an FEC assembly fluidly connected to a house-vacuum system in accordance with some embodiments.
FIG. 4B provides an FEC assembly fluidly connected to a standalone vacuum pump in accordance with some embodiments.
FIG. 5A provides an MEC kit.
FIG. 5B provides an MEC kit.
FIG. 6A provides an FEC kit in accordance with some embodiments.
FIG. 6B provides an FEC kit in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "front," "back," "top," "bottom," "clockwise," "counter clockwise," or other similar terms are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

Unless defined otherwise, all technical and scientific terms used herein have the same meaning as commonly understood by those of ordinary skill in the art.

Hospitals around the country are understandably focusing their efforts on reducing risk of CAUTIs in response to at least large penalties put in place by the ACA. Steps taken to reduce the risk of such indwelling catheter-associated infections include implementation of a variety of prevention methodologies recommended by the CDC, as well as decreasing utilization of indwelling catheters through use of non-indwelling catheter alternatives. With the use of non-indwelling catheter alternatives such as external catheters expected to rise, problems associated with application and management of such catheters once applied are also expected to rise.

Disclosed herein are external catheters, external catheter-related components, and external-catheter kits with step-by-step instructions, providing a standardized system for application and management of such catheters.

### Male External Catheter (MEC)

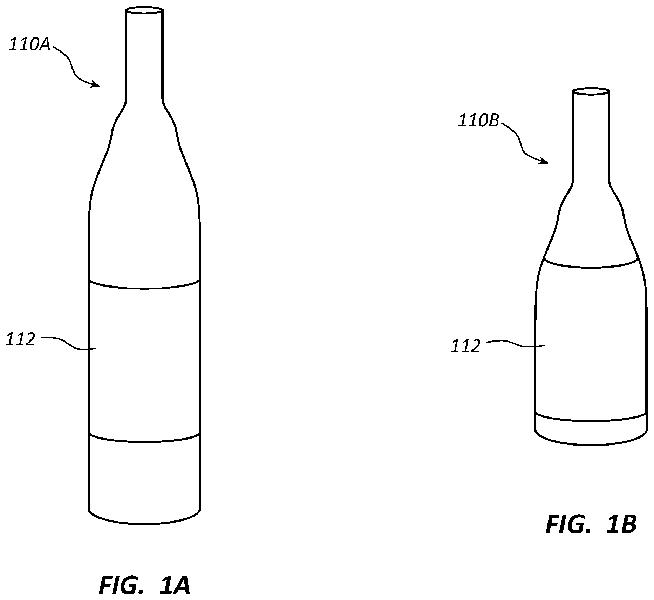

FIGS. 1A-1D show a number of MECs. Such MECs can have sheaths of latex, non-latex such as silicone, or non-latex and non-silicone such as polyurethane. Non-latex sheaths such as silicone sheaths provide breathability over latex sheaths. Such MECs can also include adhesive for self-adhering sheaths. MECs without self-adhering sheaths use adjustable straps over the MECs in lieu of adhesive.

FIG. 1A provides an unrolled MEC 110A of a first style.

As shown, the MEC 110A has a more traditional sheath length (e.g., a sheath length of about 3") and placement of a band of adhesive 112 along a length of a silicone sheath. The adhesive can be a hydrocolloid adhesive integrated into a lining of the sheath about a medial section of the MEC 110A. A width of the band of the adhesive 112 can be about 1.75" along the medial section of the MEC 110A. The combination of the silicon sheath and the hydrocolloid adhesive provides a supple, breathable MEC 110A that wicks away moisture when worn.

FIG. 1B provides an unrolled MEC 110B of a second style.

As shown, the MEC HOB has a shorter sheath length (e.g., a sheath length of about 1.5") and forward placement of the band of adhesive 112 along a length of a silicone sheath. The adhesive can be a hydrocolloid adhesive integrated into a lining of the sheath about a medial section of the MEC HOB. The width of the band of the adhesive 112 can be about 1.75" along the medial section of the MEC 110B, which provides a maximum amount of adhesive over the length of the sheath. The combination of the silicon sheath and the hydrocolloid adhesive provides a supple, breathable MEC 110B that wicks away moisture when worn.

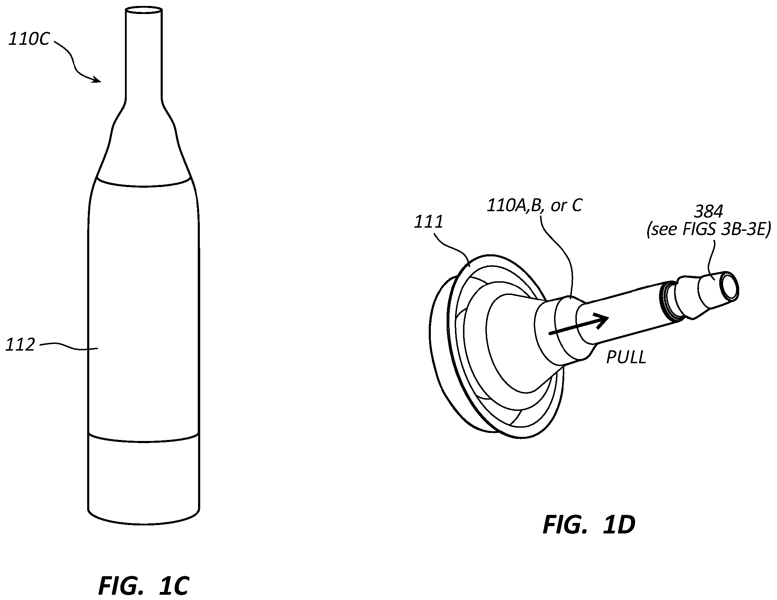

FIG. 1C provides an unrolled MEC HOC of a third style.

As shown, the MEC HOC has a more traditional sheath length (e.g., a sheath length of about 3") and forward placement of a band of adhesive 112 along a length of a silicone sheath. The adhesive can be a hydrocolloid adhesive integrated into a lining of the sheath about a medial section of the MEC HOC. The width of the band of the adhesive 112 can be about 3.00" along the medial section of the MEC HOC, which provides a maximum amount of adhesive over the length of the sheath compared to the MEC 110A. The combination of the silicon sheath and the hydrocolloid adhesive provides a supple, breathable MEC HOC that wicks away moisture when worn.

FIG. 1D provides a rolled MEC such as any MEC of MECs, 110A, 110B, and 110C disposed over a plastic insert 111.

As shown, a rolled MEC such as any MEC of MECs, 110A, 110B is disposed over the plastic insert 111 to maintain structural integrity of the MEC prior to use. Any MEC described herein can be packaged with such a plastic insert, which is easily removed by pulling the MEC away from the plastic insert as shown.

Each MEC of the MECs 110A, 110B, and 110C is manufactured with an inner diameter selected from 25 mm ("small"), 29 mm ("medium"), 32 mm ("intermediate"), 36 mm ("large"), and 41 mm ("extra-large") to ensure a proper fit.

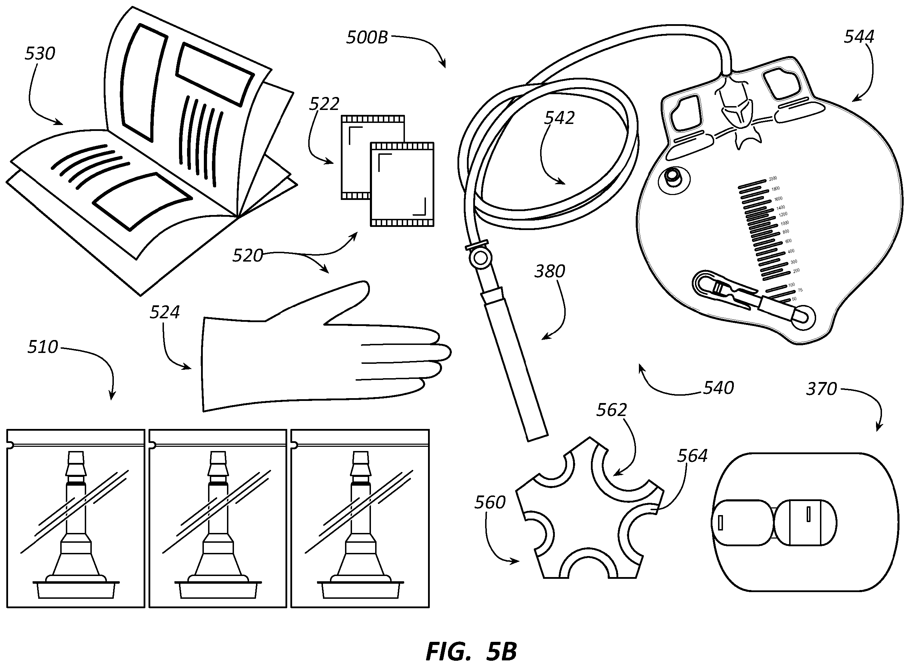

Each MEC of the MECs 110A, 110B, and HOC of a same size can be manufactured with a common color by way of a dye added to the raw material for manufacturing the same-sized MECs. As set forth herein, each MEC of the MECs 110A, 110B, and 110C can include a flexible connector 384, which flexible connector 384 can have the same color as the MEC into which it is inserted. The color is part of a color-based MEC size-indicator system in which each size of MEC has a different color, optionally in accordance with an MEC sizer. *(See* the MEC sizer 560 of FIG. 5B.) Alternatively, an MEC attachment colored in accordance with the color-based MEC size-indicator system can be used to indicate an associated MEC size. For example, a colored band can be placed around an MEC or one or more MEC-related components to indicate an associated MEC size.

The hydrocolloid adhesive of each MEC of the MECs 110A, 110B, and HOC maintains adhesion while maintaining skin integrity without skin maceration.

### Female External Catheter (FEC)

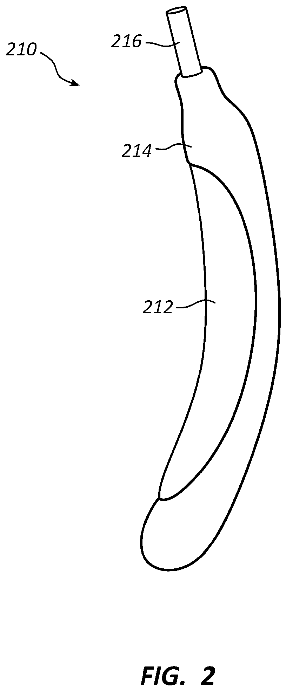

FIG. 2 provides an FEC 210 in accordance with some embodiments.

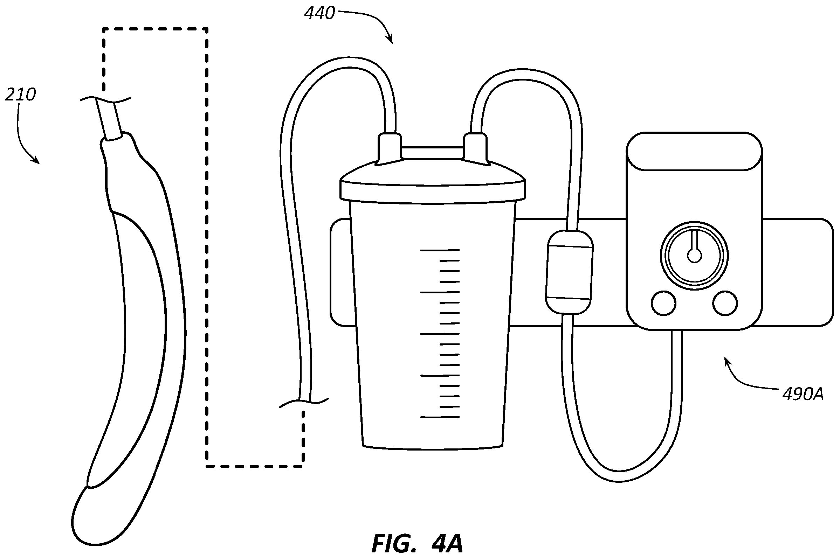

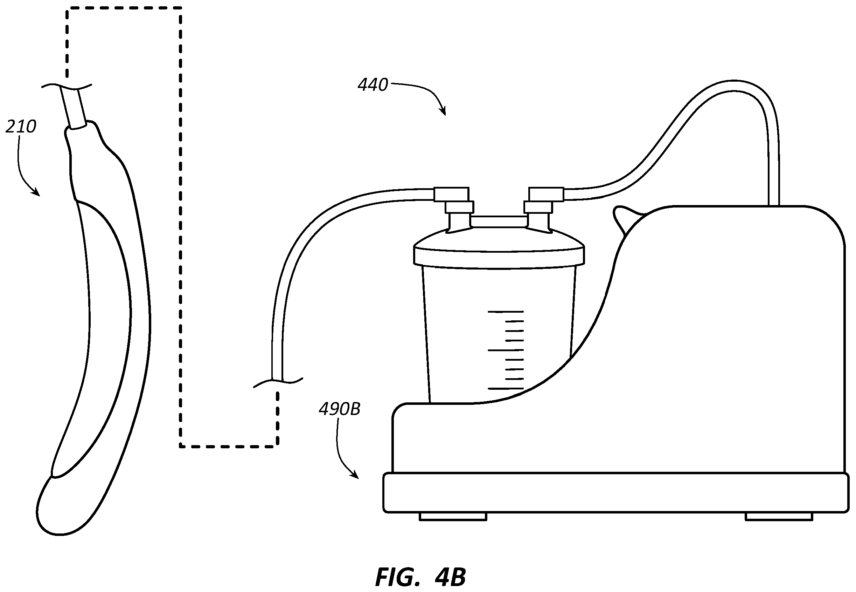

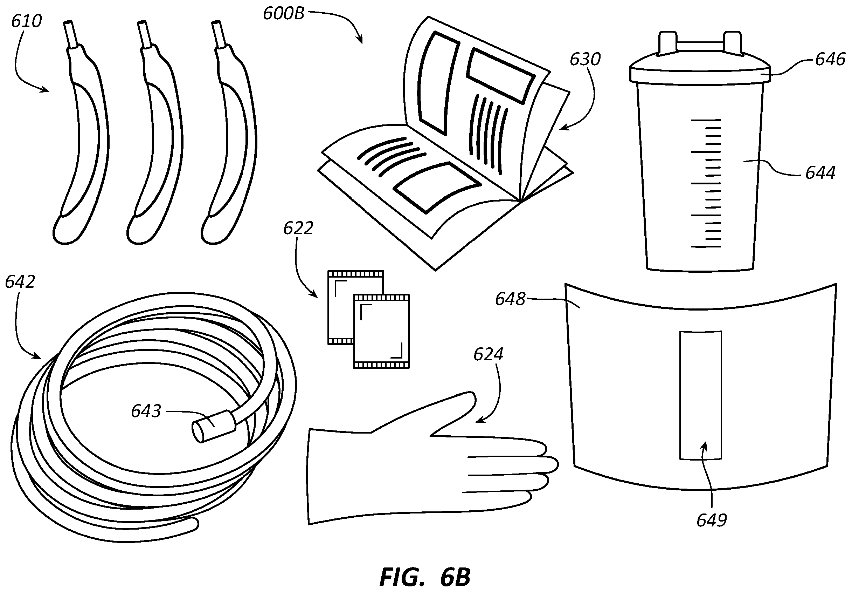

As shown, the FEC 210 includes gauze 212 covering an aperture in an elongate body 214. The aperture is fluidly connected to urine-drainage tubing 216, which is configured for connection to urine-drainage tubing of a urine-drainage system. *(See* the urine-drainage tubing 642 of FIG. 6B and the urine-drainage system 440 of FIGS. 4A and 4B.) The gauze 212 is configured to collect urine over a length and width of the aperture in the elongate body 214, and when the FEC 210 is connected to a light vacuum source (e.g., 40 mm Hg), the urine is wicked away through the aperture and the urine-drainage tubing 216.

While the FEC 210 is primarily configured for female external catheterization, there is a need for a vacuum-assisted external catheter such as the FEC 210 for small, buried (i.e., hidden beneath anatomical structures such as the abdomen, thigh, or scrotum), or inverted male anatomies. As such, the FEC 210, or a modified version thereof, can also be used for male external catheterization if needed.

### MEC-Related Components

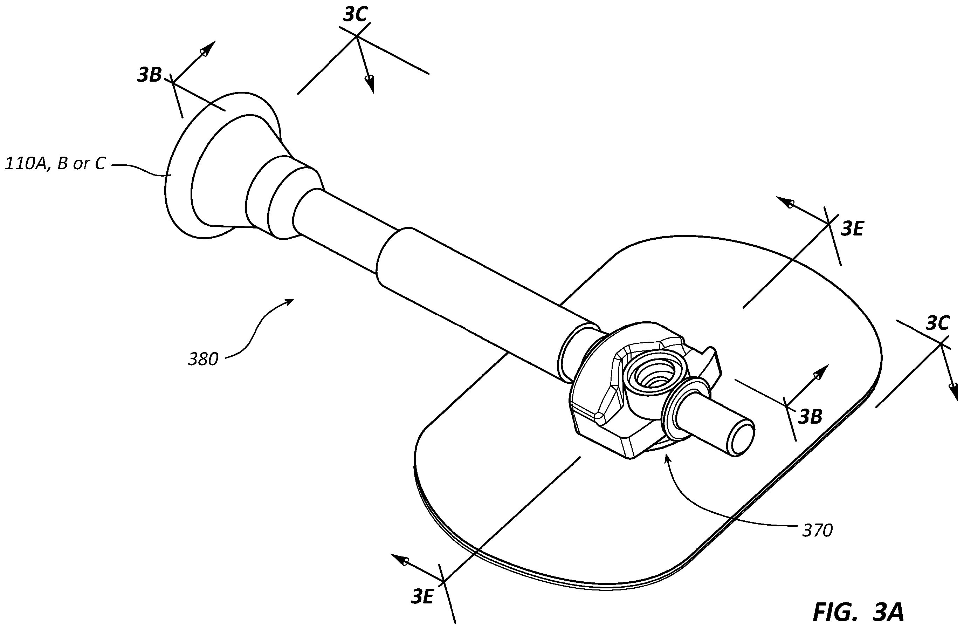

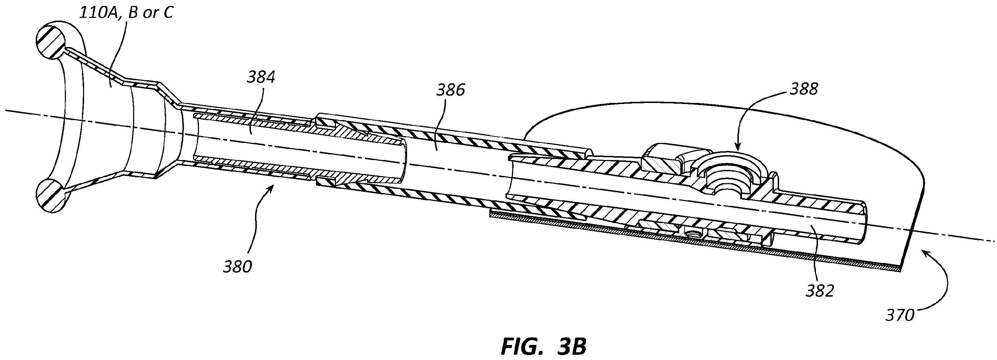

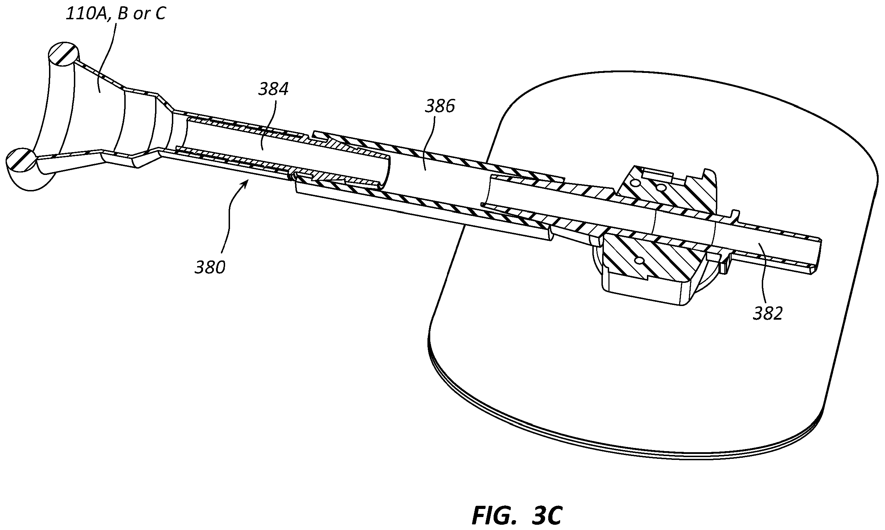

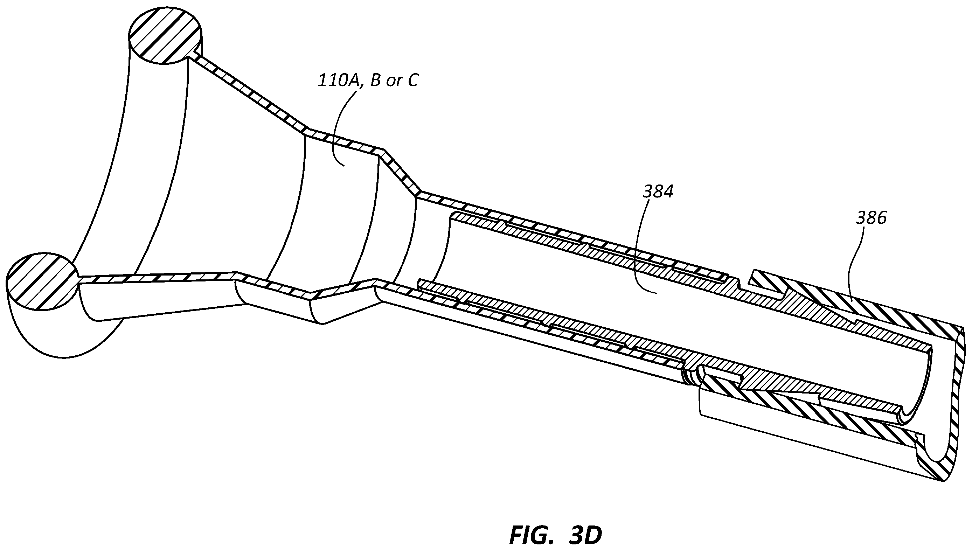

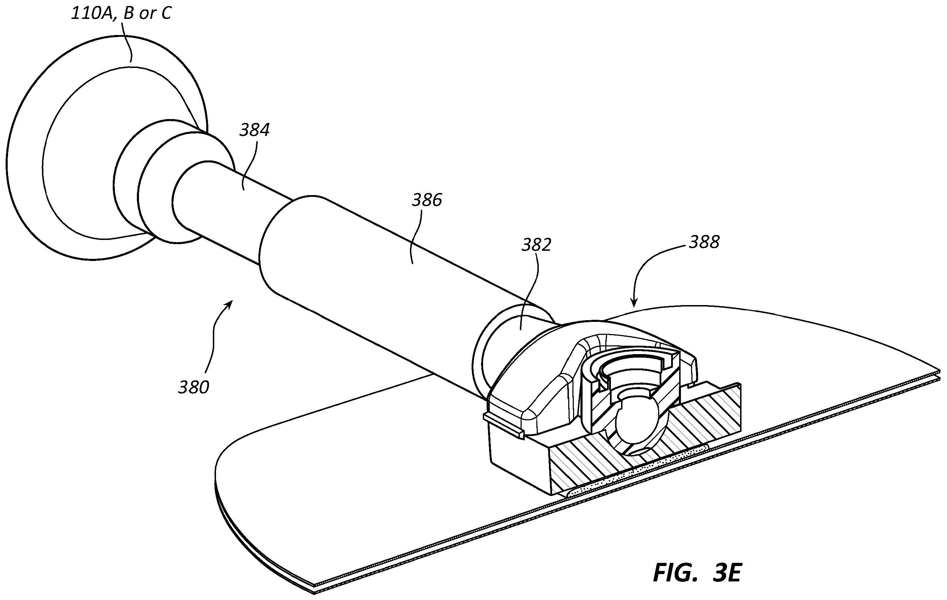

FIG. 3A provides a connector assembly 380 for an MEC such as the MEC 110A, HOB, or HOC disposed in a securement device 370. FIGS. 3B, 3C, and 3E provide various cross-sections of the connector assembly 380 assembly disposed in the securement device 370. FIG. 3D provides a close-up of a portion of the connector assembly 380 of FIG.3B.

As shown, the connector assembly 380 includes a securable tubing connector 382 and a flexible, tubular bridge 386 between the securable tubing connector 382 and a flexible tubing connector 384 of an MEC when the connector assembly 380 is assembled. The securable tubing connector 382 includes a urine-sampling port 388.

Each tubing connector of the securable tubing connector 382 and the flexible tubing connector 384 is a bidirectional tubing connector. Each tubing connector of the securable tubing connector 382 and the flexible tubing connector 384 also has a tapered end portion configured to insert in an opposing end portion of the flexible bridge 386. The securable tubing connector 382 has a non-tapered end portion configured to insert in an end portion of urine-drainage tubing of a urine-drainage system. The flexible tubing connector 384 has a non-tapered end portion configured to insert in an open-ended portion of any MEC configured for urine drainage.

The flexible tubing connector 384 and the flexible bridge 386, together, are configured for comfortably and fluidly connecting an MEC to a urine-drainage system making the connector assembly 380 part of the urine-drainage system. *(See* urine-drainage system 540 of FIG. 5B.) The flexible tubing connector 384 can have a number of circumferential ridges on the non-tapered end portion configured to provide resistance to an MEC from slipping off the non-tapered end portion while the MEC is in use.

The securable tubing connector 382 is also configured for placement in a human body-adherable securement device such as the securement device 370. The securement device 370 can include, but is not limited to, a StatLock^{®} device (C. R. Bard, Inc., Murray Hill, New Jersey, USA) or the like.

### FEC-Related Components

FIG. 4A provides the FEC 210 fluidly connected to a house-vacuum system 490A in accordance with some embodiments. FIG. 4B provides the FEC 210 fluidly connected to a standalone vacuum pump 490B in accordance with some embodiments.

As shown, the FEC 210 is fluidly connected to a light vacuum source (e.g., 40 mm Hg) such as the house-vacuum system 490A or the standalone vacuum pump 490B by way of a urine-drainage system 440. The urine-drainage system includes a urine-collection canister 644, a lid 646 configured to cover the urine-collection canister 644, and urine-drainage tubing 642 as shown and described in reference to FIG. 6B. When the FEC 210 is connected to the light vacuum source, urine can be wicked away through the aperture and the urine-drainage tubing 216 of the FEC 210.

### External-Catheter Kits

External-catheter kits can include starter kits and supplemental kits, which external-catheter kits standardize external catheterization and minimize procedural variation for better outcomes. Starter kits generally include most, if not all, single-use and multi-use components needed for an anatomical cleansing, an external-catheter application, and some external-catheter management subsequent to the external-catheter application. Supplemental kits generally include single-use components to supplement the commonly used single-use components of the starter kits. That said, due to the different styles and sizes of MECs, a starter kit for external male catheterization optionally includes one or more MECs. When such a starter kit does not include one or more MECs, a supplemental kit including the one or more MECs complements and completes the starter kit. Such starter and supplemental kits are described in more detail below.

Beginning with supplemental kits for external catheterization, such external-catheter kits include, in some embodiments, one or more external catheters, a cleansing kit, and step-by-step instructions for using the external-catheter kit. The cleansing kit includes one or more individually wrapped towelettes (e.g., castile soap wipes) for at least anatomical cleansing before application of any external catheter of the one or more external catheters. The cleansing kit optionally further includes one or more pairs of exam gloves for at least the anatomical cleansing. The step-by-step instructions for using the external-catheter kit include step-by-step instructions for the anatomical cleansing, external-catheter application, and external-catheter management subsequent to the external-catheter application.

Adverting to starter kits for external catheterization, such external-catheter kits further include (i.e., in addition to the components of the supplemental kits) a urine-drainage system. The urine-drainage system includes a urine-collection receptacle, urine-drainage tubing, and one or more connectors to fluidly connect an external catheter of the one or more external catheters to the urine-collection device. The urine-drainage system is at least partially preassembled in the external-catheter kit to minimize misconnections and misconnection-based contamination, which can result from mishandling components of the urine-drainage system. The urine-drainage tubing is fluidly connected to the urine-collection receptacle in the partially preassembled urine-drainage system.

Whether a starter kit or a supplemental kit for external catheterization, the step-by-step instructions for using the external-catheter kit are provided in a booklet having a picture book format. A booklet allows the step-by-step instructions to be conveniently set at some distance away from the external-catheter kit. Alternatively, the step-by-step instructions are integrated with packaging for the external-catheter kit. Optionally, the integrated instructions are revealed as one or more components of the external-catheter kit are removed from the external-catheter kit. Step-by-step instructions in a combination of a booklet and integrated with the packaging can also be provided in the external-catheter kit. Labels for components of the external-catheter kit are used in conjunction with the step-by-step instructions. Such labels can be on the components or packaging for the components.

The packaging of either a starter kit or a supplemental kit for external catheterization can include a wrap of a first sterilizable material (e.g., Central Supply Room ["CSR"] wrap) around the components of the external-catheter kit. Alternatively, the packaging can include a tray of a second sterilizable material (e.g., a moldable plastic) into which the components of the external-catheter kit are placed. The labels for the components of the external-catheter kit used in conjunction with the step-by-step instructions can be on the wrap or the tray. The packaging can further include an outer packaging around the wrap or the tray. The outer packaging can be configured for sterilizing the components of the external-catheter kit in the wrap or tray within the outer packaging with a sterilizing gas before sealing the external-catheter kit. That said, the external-catheter kit need not be sterilized.

### Male External-Catheter Kits

Again, external-catheter kits, which include MEC kits, can include starter kits and supplemental kits. Starter kits for male external catheterization generally include most, if not all, single-use and multi-use components needed for an anatomical cleansing, an MEC application, and some external-catheter management subsequent to the MEC application. Supplemental kits for male external catheterization generally include single-use components to supplement the commonly used single-use components of the starter kits. That said, due to the different styles and sizes of MECs, a starter kit for external male catheterization optionally includes one or more MECs. When such a starter kit does not include one or more MECs, a supplemental kit including the one or more MECs complements and, therefore, completes the starter kit. Such starter and supplemental kits are described in more detail below.

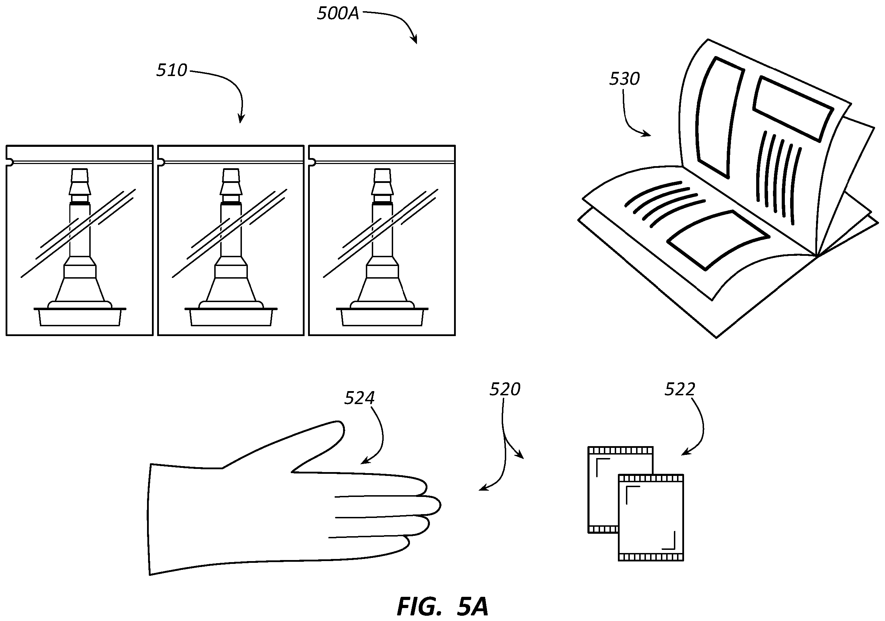

Beginning with supplemental kits for male external catheterization, FIG. 5A provides such an MEC kit 500A.

As shown, the MEC kit 500A includes one or more MECs 510, a cleansing kit 520, and step-by-step instructions 530 for using the MEC kit 500A. The one or more MECs 510 are optionally individually wrapped. The cleansing kit 520 includes one or more individually wrapped towelettes 522 (e.g., castile soap wipes) for at least anatomical cleansing before application of any MEC of the one or more MECs 510. The cleansing kit 520 optionally further includes one or more pairs of exam gloves 524 for at least the anatomical cleansing. The step-by-step instructions 530 for using the MEC kit 500A include instructions for the anatomical cleansing, MEC application, and MEC management subsequent to the MEC application.

Each MEC of the one or more MECs 510 of the MEC kit 500A has a same size and color. Each MEC of the MECs 510 can include the flexible connector 384, which flexible connector 384 can have the same color as the MEC into which it is inserted. The color is part of a color-based MEC size-indicator system in which each size of MEC has a different color, optionally in accordance with an MEC sizer. Alternatively, an MEC attachment colored in accordance with the color-based MEC size-indicator system can be used to indicate an associated MEC size. For example, a colored band can be placed around an MEC or one or more MEC-related components to indicate an associated MEC size. The packaging of the MEC kit 500A, which is described in more detail below, includes either a wrap or a tray, outer packaging, or a combination thereof (e.g., outer packaging around the wrap or the tray). Such packaging can match the color of the one or more MECs 510 (or attachments) in accordance with the color-based MEC size-indicator system.

Adverting to starter kits for male external catheterization, FIG. 5B provides such an MEC kit 500B.

As shown, the MEC kit 500B includes one or more, optionally, individually wrapped, MECs 510; however, such MECs are optional in starter kits for male external catheterization due to the different styles and sizes of MECs. When such a starter kit includes the one or more MECs 510, each MEC of the one or more MECs 510 has a different size. Furthermore, each MEC of the one or more MECs 510 can have a different color in accordance with a color-based MEC size-indicator system. (Note that each MEC of the one or more MECs 510 of the MEC kit 500A has a same size and color.) Alternatively, an MEC attachment colored in accordance with the color-based MEC size-indicator system can be used to indicate an associated MEC size. For example, a colored band can be placed around an MEC or one or more MEC-related components to indicate an associated MEC size. The one or more MECs 510 respectively represent at least the most common MEC size or sizes. That said, the one or more MECs can be five differently sized MECs corresponding to an MEC sizer 560.

The MEC kit 500B further includes (i.e., in addition to the components of the MEC kit 500A) an MEC sizer 560. The MEC sizer 560 has up to five differently sized cutouts for determining an appropriate MEC size for a user or patient. Each cutout 562 of the cutouts of the MEC sizer 560 can have a differently colored border 564 around the cutout 562 in accordance with a color-based MEC size-indicator system. The MEC sizer 560 can be used to select an MEC of the one or more differently sized MECs 510, if present, or the MEC sizer 560 can be used to select a supplemental kit for male external catheterization such as the MEC kit 500A.

The MEC kit 500B further includes a urine-drainage system 540. The urine-drainage system 540 includes a urine-collection bag 544, urine-drainage tubing 542, and a connector assembly 380 to fluidly connect an MEC to the urine-collection bag 544. The urine-collection bag 544 is a bedside bag or a leg bag.

The connector assembly 380 includes a securable tubing connector 382 having a urine-sampling port 388 and a flexible, tubular bridge 386 configured to connect to a flexible tubing connector 384 of an MEC. *(See* FIGS. 3A-3E.) The connector assembly 380 is configured by way of the flexible bridge 386 to comfortably connect to a flexible tubing connector 384 of an MEC of the one or more MECs 510, thereby fluidly connecting the MEC to the urine-collection bag 544 by way of the urine-drainage tubing 542. Each tubing connector of the securable tubing connector 382 and the flexible tubing connector 384 is a bidirectional tubing connector. Each tubing connector of the securable tubing connector 382 and the flexible tubing connector 384 also has a tapered end portion configured to insert in an opposing end portion of the flexible bridge 386. The securable tubing connector 382 has a non-tapered end portion configured to insert in an end portion of the urine-drainage tubing 542. The flexible tubing connector 384 has a non-tapered end portion configured to insert in an end portion of any MEC configured for urine drainage.

The urine-drainage system 540 is at least partially preassembled in the MEC kit 500B to minimize misconnections and misconnection-based contamination. The urine-drainage tubing 542 fluidly connects the connector assembly 380 to the urine-drainage bag 544 in the partially preassembled urine-drainage system 540.

The MEC kit 500B further includes a securement device 370 configured to secure the securable tubing connector 382 of the connector assembly 380 to a patient's leg. The securement device 370 minimizes any longitudinal or transverse movement of the urine-drainage tubing 542 while in use. Such a securement device can include, but is not limited to, a StatLock^{®} device (C. R. Bard, Inc., Murray Hill, New Jersey, USA) or the like.

Whether a starter kit or a supplemental kit for male external catheterization, the step-by-step instructions for using the MEC kit are provided in a booklet having a picture book format. A booklet allows the step-by-step instructions to be conveniently set at some distance away from the MEC kit. Alternatively, the step-by-step instructions are integrated with packaging for the MEC kit. Optionally, the integrated instructions are revealed as one or more components of the MEC kit are removed from the MEC kit. Step-by-step instructions in a combination of a booklet and integrated with the packaging can also be provided in the MEC kit. Labels for components of the MEC kit are used in conjunction with the step-by-step instructions. Such labels can be on the components or packaging for the components.

The step-by-step instructions, particularly those in starter kits for male external catheterization, are further for identifying supplemental kits for male external catheterization that include one or more supplemental MECs in accordance with the color-based MEC size- indicator system. The supplemental kits including the one or more supplemental MECs thereof and at least some packaging of the supplemental kits are individually colored to match the differently colored borders of the cutouts of the MEC sizer 560 in accordance with the color-based MEC size-indicator system.

The packaging of either a starter kit or a supplemental kit for male external catheterization can include a wrap of a first sterilizable material (e.g., CSR wrap) around the components of the MEC kit. Alternatively, the packaging can include a tray of a second sterilizable material (e.g., a moldable plastic) into which the components of the MEC kit are placed. The labels for the components of the MEC kit used in conjunction with the step-by-step instructions can be on the wrap or the tray. The packaging can further include an outer packaging around the wrap or the tray. The outer packaging can be configured for sterilizing the components of the MEC kit in the wrap or tray within the outer packaging with a sterilizing gas before sealing the MEC kit. That said, the MEC kit need not be sterilized.

While not shown in either FIG. 5A or 5B, a starter kit or a supplemental kit for male external catheterization can further include a visual duration indicator configured to indicate when a predetermined amount of time has elapsed since connecting an MEC to the urine-collection bag 544. This is useful for reminding a user or patient to replace the MEC after the predetermined amount of time has elapsed. The predetermined amount of time can be at least 4 hours, 8 hours, 12 hours, 16 hours, 20 hours, or 24 hours. The predetermined amount of time can be no more than 24 hours, 20 hours, 16 hours, 12 hours, 8 hours, or 4 hours. As such, the predetermined amount of time can be at least 4 hours and no more than 24 hours, including at least 8 hours and no more than 20 hours, such as at least 8 hours and no more than 16 hours, or, for example, about 12 hours.

The visual duration indicator is configured for activation and, thereby, starting the predetermined amount of time during one or more steps of a procedure for connecting the MEC to the urine-collection bag 544. Activation can include, but is not limited to, peeling an adhered backing off a component of MEC kit 500A or 500B or breaking a fluid-filled blister on a component of MEC kit 500A or 500B (e.g., an MEC attachment colored in accordance with the color-based MEC size-indicator system) to start a process of chemical migration, chemical reaction, or a combination thereof resulting in a discernable visual endpoint indicating the predetermined amount of time has elapsed. Subsequent to activation of the visual duration indicator prior to or at the time of connecting the MEC to the urine-collection bag 544, the visual duration indicator will change color (e.g., lighter color, different color, no color) after the predetermined amount of time has elapsed reminding the user or patient to replace the MEC.

### Female External-Catheter Kits

Again, external-catheter kits, which include FEC kits, can include starter kits and supplemental kits. Starter kits for female external catheterization generally include most, if not all, single-use and multi-use components needed for an anatomical cleansing, an FEC application, and some external-catheter management subsequent to the FEC application. Supplemental kits for female external catheterization generally include single-use components to supplement the commonly used single-use components of the starter kits.

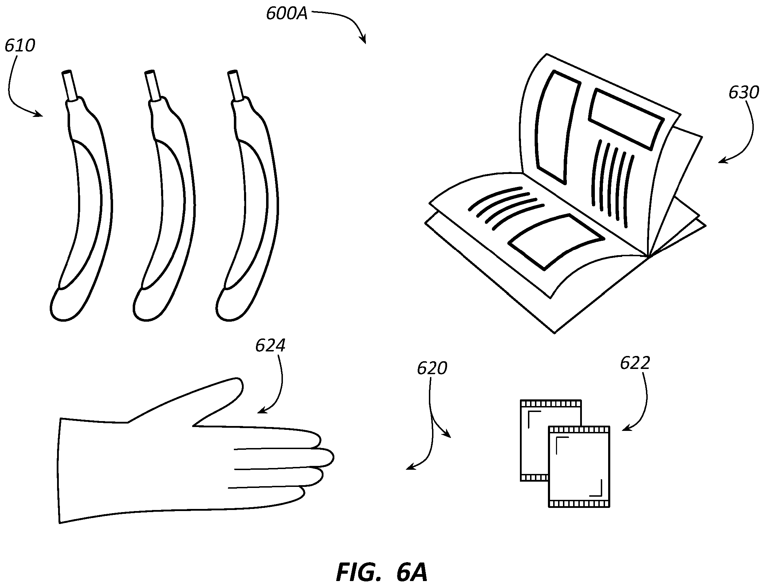

Beginning with supplemental kits for female external catheterization, FIG. 6A provides such an FEC kit 600A in accordance with some embodiments.

As shown, the FEC kit 600A includes one or more FECs 610, a cleansing kit 620, and step-by-step instructions 630 for using the FEC kit 600A. The one or more FECs 610 are optionally individually wrapped. The cleansing kit 620 includes one or more individually wrapped towelettes 622 (e.g., castile soap wipes) for at least anatomical cleansing before application of any FEC of the one or more FECs 610. The cleansing kit 620 optionally further includes one or more pairs of exam gloves 624 for at least the anatomical cleansing. The step-by-step instructions 630 for using the FEC kit 600A include instructions for the anatomical cleansing, FEC application, and FEC management subsequent to the FEC application.

Adverting to starter kits for female external catheterization, FIG. 6B provides such an FEC kit 600B in accordance with some embodiments.

As shown, the FEC kit 600B further includes (i.e., in addition to the components of the FEC kit 600A) a urine-drainage system 440 *(see* FIGS. 4A and 4B). The urine-drainage system 440 includes a urine-collection canister 644, a lid 646 configured to cover the urine-collection canister 644, urine-drainage tubing 642, and one or more connectors such as connector 643 to fluidly connect an FEC of the one or more FECs 610 to the urine-collection canister 644.

The urine-drainage system 440 is at least partially preassembled in the FEC kit 600B to minimize misconnections and misconnection-based contamination. The urine-drainage tubing 642 is fluidly connected to the urine-collection canister 644 through the lid 646 covering the urine-collection canister 644 in the partially preassembled urine-drainage system 440.

The FEC kit 600B further includes a urine-privacy cover 648 configured to form a sleeve for covering the urine-collection canister 644. The sleeve has a cut-out window 649 configured to expose graduations on the urine-collection canister 644 through the window 649 of the sleeve while a remainder of the urine-collection canister 644 is covered by the sleeve.

While not shown, the FEC kit 600B can further include a securement device configured to secure the urine-drainage tubing 642 of the urine-drainage system 440 to a patient's leg or another portion of the patient's body. The securement device minimizes any longitudinal or transverse movement of the urine-drainage tubing 642 while in use. Such a securement device can include, but is not limited to, a StatLock^{®} device (C. R. Bard, Inc., Murray Hill, New Jersey, USA) or the like.

Whether a starter kit or a supplemental kit for female external catheterization, the step-by-step instructions for using the FEC kit are provided in a booklet having a picture book format. A booklet allows the step-by-step instructions to be conveniently set at some distance away from the FEC kit. Alternatively, the step-by-step instructions are integrated with packaging for the FEC kit. Optionally, the integrated instructions are revealed as one or more components of the FEC kit are removed from the FEC kit. Step-by-step instructions in a combination of a booklet and integrated with the packaging can also be provided in the FEC kit. Labels for components of the FEC kit are used in conjunction with the step-by-step instructions. Such labels can be on the components or packaging for the components.

The packaging of either a starter kit or a supplemental kit for female external catheterization can include a wrap of a first sterilizable material (e.g., CSR wrap) around the components of the FEC kit. Alternatively, the packaging can include a tray of a second sterilizable material (e.g., a moldable plastic) into which the components of the FEC kit are placed. The labels for the components of the FEC kit used in conjunction with the step-by-step instructions can be on the wrap or the tray. The packaging can further include an outer packaging around the wrap or the tray. The outer packaging can be configured for sterilizing the components of the FEC kit in the wrap or tray within the outer packaging with a sterilizing gas before sealing the FEC kit. That said, the FEC kit need not be sterilized.

While not shown in either FIG. 6A or 6B, a starter kit or a supplemental kit for female external catheterization can further include a visual duration indicator configured to indicate when a predetermined amount of time has elapsed since connecting an FEC to the urine-collection canister 644. This is useful for reminding a user or patient to replace the FEC after the predetermined amount of time has elapsed. The predetermined amount of time can be at least 4 hours, 8 hours, 12 hours, 16 hours, 20 hours, or 24 hours. The predetermined amount of time can be no more than 24 hours, 20 hours, 16 hours, 12 hours, 8 hours, or 4 hours. As such, the predetermined amount of time can be at least 4 hours and no more than 24 hours, including at least 8 hours and no more than 20 hours, such as at least 8 hours and no more than 16 hours, or, for example, about 12 hours.

The visual duration indicator is configured for activation and, thereby, starting the predetermined amount of time during one or more steps of a procedure for connecting the FEC to the urine-collection canister 644. Activation can include, but is not limited to, peeling an adhered backing off a component of FEC kit 600A or 600B or breaking a fluid-filled blister on a component of FEC kit 600A or 600B to start a process of chemical migration, chemical reaction, or a combination thereof resulting in a discernable visual endpoint indicating the predetermined amount of time has elapsed. Subsequent to activation of the visual duration indicator prior to or at the time of connecting the FEC to urine-collection canister 644, the visual duration indicator will change color after the predetermined amount of time has elapsed reminding the user or patient to replace the FEC.

### Methods

Exemplary methods of external catheters, external catheter-related components, or external-catheter kits include methods of making or using the external catheters, external catheter-related components, or external-catheter kits. For example, methods of making the external-catheter kits include kitting the components of the external-catheter kits, optionally sterilizing the components of the external-catheter kits, and sealing the external-catheter kits. For example, methods of using the external-catheter kits include opening the external-catheter kits and following the step-by-step instructions provided in the external-catheter kits, thereby following a standardized system for application and management of external catheters with minimized procedural variation.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. An external-catheter kit, comprising:
one or more external catheters (210, 610), wherein the one or more external catheters are female external catheters ("FECs") (210, 610), wherein the FEC (210, 610) comprises an elongate body (214) and gauze (212) covering an aperture in said elongate body (214); wherein said aperture is fluidly connected to a urine-drainage tubing (216), wherein said urine-drainage tubing (216) is configured for connection to an additional urine-drainage tubing (642) of a FEC urine-drainage system (440);
a cleansing kit (620) including one or more individually wrapped towelettes (622) for at least anatomical cleansing before application of any external catheter of the one or more external catheters; and
step-by-step instructions (630) for the anatomical cleansing, external-catheter application, and external-catheter management subsequent to the external-catheter application.

2. The external-catheter kit of claim 1, wherein the one or more FECs (210, 610) are individually wrapped.

3. The external-catheter kit of any claim of claims 1-2, comprising a FEC urine-drainage system (440) including a urine-collection canister (644), a lid (646) configured to cover the urine-collection canister, the additional urine-drainage tubing (642), and one or more connectors (643) to fluidly connect an FEC of the one or more FECs to the urine-collection canister (644).

4. The external-catheter kit of claim 3, wherein the FEC urine-drainage system (440) is at least partially preassembled in the external-catheter kit to minimize misconnections and misconnection-based contamination, and wherein the additional urine-drainage tubing (642) is fluidly connected to the urine-collection canister (644) through the lid (646) covering the urine-collection canister (644) in the partially preassembled FEC urine-drainage system.

5. The external-catheter kit of claim 4, further comprising a urine-privacy cover (648) configured to form a sleeve for covering the urine-collection canister (644), wherein the sleeve has a cut-out window (649) configured to expose graduations on the urine-collection canister (644) through the window (649) of the sleeve while a remainder of the urine-collection canister (644) is covered by the sleeve.

6. The external-catheter kit of any claim of claims 1-5, further comprising a visual duration indicator configured to indicate when a predetermined amount of time has elapsed since activation thereof.

7. The external-catheter kit of any claim of claims 1-6, further comprising a securement device configured to secure the additional urine-drainage tubing (642) of the urine-drainage system (440) to a patient's leg or another portion of the patient's body.

8. The external-catheter kit of any of claims 1-7, wherein the step-by-step instructions are provided in a booklet having a picture book format, integrated with packaging for the external-catheter kit, optionally, revealed as one or more components of the external-catheter kit are removed from the external-catheter kit, or a combination thereof.

9. The external-catheter kit of claim 8, wherein the packaging includes a wrap of a first sterilizable material around the components of the external-catheter kit or a tray of a second sterilizable material into which the components of the external-catheter kit are placed.

10. The external-catheter kit of claim 9, wherein the packaging further includes an outer packaging around the wrap or the tray.

11. The external-catheter kit of claim 10, wherein the outer packaging is configured for sterilizing the components of the external-catheter kit in the wrap or tray within the outer packaging with a sterilizing gas before sealing the external-catheter kit.

## Patentansprüche

1. Außenkatheter-Kit, umfassend:
einen oder mehrere Außenkatheter (210, 610), wobei der eine oder die mehreren Außenkatheter weibliche Außenkatheter ("FECs") (210, 610) sind, wobei der FEC (210, 610) einen länglichen Körper (214) und Gaze (212) umfasst, die eine Öffnung in dem länglichen Körper (214) bedeckt; wobei die genannte Öffnung strömungstechnisch zu einem Urindrainageschlauch (216) verbunden ist, wobei der besagte Urindrainageschlauch (216) konfiguriert ist zur Verbindung mit einem zusätzlichen Urindrainageschlauch (642) eines FEC-Urindrainagesystems (440);
ein Reinigungsset (620) einschließlich eines oder mehrerer einzeln verpackter Feuchttücher (622) für mindestens eine anatomische Reinigung vor der Anwendung eines beliebigen Außenkatheters des einen oder der mehreren Außenkatheter; und
schrittweise Anleitungen (630) für die anatomische Reinigung, die Anlage eines Außenkatheters und das Management des Außenkatheters nach der Anlage des Außenkatheters.

2. Außenkatheter-Kit nach Anspruch 1, wobei der eine oder die mehreren FECs (210, 610) einzeln umhüllt sind.

3. Außenkatheter-Kit nach einem der Ansprüche 1-2,
umfassend ein FEC-Urindrainagesystem (440) einschließlich eines Urinsammelkanisters (644), eines Deckels (646), der konfiguriert ist, den Urinsammelkanister zu bedecken, den zusätzlichen Urindrainageschlauch (642) und einen oder mehrere Verbinder (643), um ein FEC der einen oder mehreren FECs fluidisch mit dem Urinsammelkanister (644) zu verbinden.

4. Außenkatheter-Kit nach Anspruch 3,
wobei das FEC-Urindrainagesystem (440) zumindest teilweise im Außenkatheter-Kit vormontiert ist, um Fehlverbindungen und auf Fehlverbindungen basierende Kontamination zu minimieren, und wobei der zusätzliche Urindrainageschlauch (642) durch den Deckel (646), der den Urinsammelkanister (644) abdeckt, fluidisch mit dem Urinsammelkanister (644) in dem teilweise vormontierten FEC-Urinableitungssystem verbunden ist.

5. Außenkatheter-Kit nach Anspruch 4,
weiterhin umfassend eine Urin-Sichtschutzabdeckung (648), die konfiguriert ist, eine Hülse zur Abdeckung des Urinsammelkanisters (644) zu bilden, wobei die Hülse ein ausgeschnittenes Fenster (649) aufweist, das dazu konfiguriert ist, Graduierungen auf dem Urinsammelkanister (644) durch das Fenster (649) der Hülse freizulegen, während ein verbleibender Teil des Urinsammelkanisters (644) von der Hülse bedeckt ist.

6. Außenkatheter-Kit nach einem der Ansprüche 1-5,
weiter umfassend einen visuellen Dauerindikator, der konfiguriert ist, anzuzeigen, wann eine vorbestimmte Zeitspanne seit dessen Aktivierung verstrichen ist.

7. Außenkatheter-Kit nach einem der Ansprüche 1-6,
weiter umfassend eine Befestigungsvorrichtung, die zum Sichern des zusätzlichen Urindrainageschlauchs (642) des Urindrainagesystems (440) an einem Bein des Patienten oder einem anderen Körperteil des Patienten konfiguriert ist.

8. Außenkatheter-Kit nach einem der Ansprüche 1-7,
wobei die schrittweisen Anleitungen in einem Heftchen bereitgestellt sind, das ein Bilderbuchformat aufweist, das in die Verpackung für das Außenkatheter-Kit integriert ist, das optional offenbart ist, wenn eine oder mehrere Komponenten des Außenkatheter-Kits aus dem Außenkatheter-Kit entfernt werden, oder eine Kombination davon.

9. Außenkatheter-Kit nach Anspruch 8,
wobei die Verpackung eine Umhüllung aus einem ersten sterilisierbaren Material um die Komponenten des Außenkatheter-Kits oder eine Schale aus einem zweiten sterilisierbaren Material einschließt, in die die Komponenten des Außenkatheter-Kits gelegt werden.

10. Außenkatheter-Kit nach Anspruch 9,
wobei die Verpackung weiter eine äußere Verpackung um die Folie oder die Schale einschließt.

11. Außenkatheter-Kit nach Anspruch 10,
wobei die äußere Verpackung konfiguriert ist zur Sterilisation der Komponenten des Außenkatheter-Kits in der Verpackung oder der Schale innerhalb der äußeren Verpackung mit einem sterilisierenden Gas vor dem Versiegeln des Außenkatheter-Kits.

## Revendications

1. Kit de cathéter externe, comprenant :
un ou plusieurs cathéters externes (210, 610), dans lequel le ou les cathéters externes sont des cathéters externes femelles (« FEC ») (210, 610), dans lequel le FEC (210, 610) comprend un corps allongé (214) et une gaze (212) couvrant une ouverture dans ledit corps allongé (214) ; dans lequel ladite ouverture est reliée de manière fluidique à un tube de drainage urinaire (216), dans lequel ledit tube de drainage urinaire (216) est configuré pour être connecté à un tube de drainage urinaire supplémentaire (642) d'un système de drainage urinaire FEC (440) ;
un kit de nettoyage (620) incluant une ou plusieurs lingettes enveloppées individuellement (622) pour au moins le nettoyage anatomique avant application du un ou plusieurs cathéters externes ; et
des instructions pas à pas (630) pour le nettoyage anatomique, l'application du cathéter externe et la gestion du cathéter externe après l'application du cathéter externe.

2. Kit de cathéter externe selon la revendication 1,
dans lequel le ou les FEC (210, 610) sont enveloppés individuellement.

3. Kit de cathéter externe selon l'une quelconque des revendications 1 à 2, comprenant un système de drainage urinaire FEC (440) incluant un récipient de collecte d'urine (644), un couvercle (646) configuré pour couvrir le récipient de collecte d'urine, le tube supplémentaire (642) de drainage d'urine et un ou plusieurs connecteurs (643) pour connecter de manière fluidique un FEC parmi les un ou plusieurs FEC au récipient de collecte d'urine (644).

4. Kit de cathéter externe selon la revendication 3,
dans lequel le système de drainage urinaire FEC (440) est au moins partiellement préassemblé dans le kit de cathéter externe afin de minimiser les erreurs de connexion et la contamination due à des erreurs de connexion, et dans lequel le tube de drainage urinaire supplémentaire (642) est relié de manière fluidique au récipient de collecte d'urine (644) à travers le couvercle (646) recouvrant le récipient de collecte d'urine (644) dans le système de drainage urinaire FEC partiellement préassemblé.

5. Kit de cathéter externe selon la revendication 4,
comprenant en outre un couvercle de protection intime de l'urine (648) configuré pour former un manchon destiné à recouvrir le récipient de collecte d'urine (644), dans lequel le manchon présente une fenêtre découpée (649) configurée pour exposer les graduations sur le récipient de collecte d'urine (644) à travers la fenêtre (649) du manchon tandis que le reste du récipient de collecte d'urine (644) est recouvert par le manchon.

6. Kit de cathéter externe selon l'une quelconque des revendications 1 à 5,
comprenant en outre un indicateur visuel de durée configuré pour indiquer quand un laps de temps prédéterminé s'est écoulé depuis son activation.

7. Kit de cathéter externe selon l'une quelconque des revendications 1 à 6,
comprenant en outre un dispositif de fixation configuré pour fixer le tube de drainage urinaire supplémentaire (642) du système de drainage urinaire (440) à une jambe du patient ou à une autre partie du corps du patient.

8. Kit de cathéter externe selon l'une quelconque des revendications 1 à 7,
dans lequel les instructions étape par étape sont fournies dans un livret au format album illustré, intégré à l'emballage du kit de cathéter externe, facultativement, révélées à mesure qu'un ou plusieurs composants du kit de cathéter externe sont retirés du kit de cathéter externe, ou une combinaison de ceux-ci.

9. Kit de cathéter externe selon la revendication 8,
dans lequel l'emballage inclut un emballage en un premier matériau stérilisable autour des composants du kit de cathéter externe ou un plateau en un second matériau stérilisable dans lequel les composants du kit de cathéter externe sont placés.

10. Kit de cathéter externe selon la revendication 9,
dans lequel l'emballage inclut en outre un emballage extérieur autour de l'enveloppe ou du plateau.

11. Kit de cathéter externe selon la revendication 10,
dans lequel l'emballage extérieur est configuré pour stériliser les composants du kit de cathéter externe dans l'enveloppe ou le plateau à l'intérieur de l'emballage extérieur avec un gaz stérilisant avant de sceller le kit de cathéter externe.
